# EUROPEAN PATENT APPLICATION

(11) **EP 4 810 032 A1**
(43) Date of publication of application: **23.09.2026**
(21) Application number: 25748111.9
(22) Date of filing: 24.01.2025
(51) Int. Cl.: A61K 9/70, A61K 47/02, A61K 47/10, A61K 47/32, A61K 47/42

(54) **CATAPLASM**

(30) Priority: 29.01.2024 JP 2024011159
(71) Applicant: Hisamitsu Pharmaceutical Co., Inc., Tosu-shi, Saga 841-0017 (JP)
(72) Inventor: SATO Sho, Tosu-shi, Saga 841-0023 (JP); TSURUSHIMA Keiichiro, Tosu-shi, Saga 841-0023 (JP); KUWAHARA Tetsuji, Tosu-shi, Saga 841-0023 (JP)
(74) Representative: Grünecker Patent- und Rechtsanwälte PartG mbB
(86) International application number: PCT/JP2025/002215
(87) International publication number: WO 2025/164529

(57) **Abstract**

This cataplasm includes a support layer, an adhesive layer laminated on the support layer, and a release liner provided on a surface of the adhesive layer which is on the opposite side from the support layer. The adhesive layer contains a drug, water, gelatin, *l*-menthol, a (methyl acrylate)-(2-ethylhexyl acrylate) copolymer resin emulsion, and magnesium aluminometasilicate, wherein the amount of the (methyl acrylate)-(2-ethylhexyl acrylate) copolymer resin emulsion is 1.5-20 mass%, the amount of magnesium aluminometasilicate is 0.25-0.45 mass%, and the amount of gelatin is 1-3 mass% relative to the whole mass of the adhesive layer.

## Description

### Technical Field

The present invention relates to a gel patch.

### Background Art

A gel patch is a type of patch produced by spreading an adhesive layer containing a drug on a support layer such as a cloth, and generally, the water content of the adhesive layer is high. In order to achieve a high water content of the adhesive layer and to secure excellent shape retention and cohesiveness, the adhesive layer includes water-soluble polymers such as gelatin, polyacrylic acid, a neutralized polyacrylic acid, and polyvinyl alcohol. In addition, a gel patch containing a cooling agent such as *l*-menthol can provide a cooling sensation at the time of use and exhibit an effect of alleviating pain and a cooling effect (for example, Patent Literature 1 and Patent Literature 2).

In a gel patch containing *l*-menthol, a problem is known in which crystals of *l*-menthol may precipitate in the adhesive layer. In order to solve this problem, for example, Patent Literature 3 suppresses precipitation of menthol by setting the content of menthol in an aqueous gel composition to a specific amount and blending at least one selected from silica and talc into the aqueous gel composition.

### Citation List

### Patent Literature

Patent Literature 1: JP 2020-132637
Patent Literature 2: JP 2019-81755
Patent Literature 3: JP 2022-75038

### Summary of Invention

### Technical Problem

In the course of developing a gel patch in which precipitation of *l-*menthol crystals is suppressed, the present inventors have found that, in addition to precipitation of *l*-menthol crystals, there may arise problems that unevenness occurs at the time of spreading, that after spreading, components of the adhesive layer seep out from the support layer, that adhesiveness and liner releasability are insufficient, and that pain occurs upon peeling off the gel patch applied to the skin.

The present invention has been made in view of the problems possessed by the above conventional techniques, and an object thereof is to provide a gel patch in which precipitation of *l*-menthol crystals is suppressed, free from spreading unevenness, adhesiveness and liner releasability are excellent, seepage is suppressed, and pain at the time of peeling is suppressed.

### Solution to Problem

As a result of extensive research conducted to achieve the above object, the present inventors have found that a gel patch with suppressed precipitation of *l*-menthol crystals can be obtained by setting the content of a methyl acrylate/2-ethylhexyl acrylate copolymer resin emulsion within a predetermined range in an adhesive layer containing water, gelatin, *l*-menthol, the emulsion, and magnesium aluminometasilicate.

However, despite the fact that the content of the methyl acrylate/2-ethylhexyl acrylate copolymer resin emulsion is set within the predetermined range, new problems have arisen in that unevenness occurs at the time of spreading of the adhesive layer, that after spreading, components of the adhesive layer seep out to the support layer, that adhesiveness and liner releasability are insufficient, and that pain occurs upon peeling off the gel patch.

As a result of intensive examination by the present inventors regarding the above problems, it has been found that the above problems can be solved by setting the amounts of magnesium aluminometasilicate and gelatin, among the components contained in the gel patch of the present invention, within predetermined ranges, and thus the present invention has been completed.

That is, the present invention includes the following [1] to [2].
[1] A gel patch comprising a support layer, an adhesive layer laminated on the support layer, and a release liner provided on a surface of the adhesive layer opposite to the support layer, wherein the adhesive layer contains a drug, water, gelatin, *l*-menthol, a methyl acrylate/2-ethylhexyl acrylate copolymer resin emulsion, and magnesium aluminometasilicate, and based on the total mass of the adhesive layer, the content of the methyl acrylate/2-ethylhexyl acrylate copolymer resin emulsion is from 1.5 % by mass to 20 % by mass, the content of the magnesium aluminometasilicate is from 0.25 % by mass to 0.45 % by mass, and the content of the gelatin is from 1 % by mass to 3 % by mass.
[2] The gel patch according to the above [1], wherein the adhesive layer further comprises at least one selected from the group consisting of polyacrylic acid and a neutralized polyacrylic acid.

### Advantageous Effects of Invention

According to the present invention, it is possible to provide a gel patch in which precipitation of *l*-menthol crystals is suppressed, free from spreading unevenness, adhesiveness and liner releasability are excellent, seepage is suppressed, and pain at the time of peeling is suppressed.

### Description of Embodiments

Hereinafter, preferred embodiments of the present invention will be described in detail.

A gel patch according to a first embodiment of the present invention comprises a support layer, an adhesive layer laminated on the support layer, and a release liner provided on a surface of the adhesive layer opposite to the support layer, wherein the adhesive layer contains a drug, water, gelatin, *l*-menthol, a methyl acrylate/2-ethylhexyl acrylate copolymer resin emulsion, and magnesium aluminometasilicate, and based on the total mass of the adhesive layer, the content of the methyl acrylate/2-ethylhexyl acrylate copolymer resin emulsion is from 1.5 % by mass to 20 % by mass, the content of the magnesium aluminometasilicate is from 0.25 % by mass to 0.45 % by mass, and the content of the gelatin is from 1 % by mass to 3 % by mass.

The support layer may be any that can support an adhesive layer containing water, and those known to persons skilled in the art can be used. Examples of the support layer include woven fabrics (including knitted fabrics), non-woven fabrics, resin films, foam sheets, and paper. When a woven fabric, a non-woven fabric, or a resin film is used as the support layer, examples of the material thereof include polyolefins such as polyethylene, polypropylene, and polybutylene, polyesters such as polyethylene terephthalate, rayon, polyurethane, and cotton. These may be used singly as one kind, or two or more kinds may be used in combination, and may be a single layer or a laminated body. The material of the support layer is more preferably a polyester.

The support layer is preferably a non-woven fabric or a woven fabric, and particularly preferably a non-woven fabric or a woven fabric having a predetermined elongation recovery rate (elastic recovery percentage of elongation). In the present specification, the "elongation recovery rate" is a value measured in accordance with JIS L1096:2010 and means an "elastic recovery percentage of elongation at constant load" or an "elastic recovery percentage of elongation during constant rate elongation". For example, a "50% elongation recovery rate" is an elastic recovery percentage of elongation at the time of elongation (constant rate elongation) such that an elongation ratio becomes 50%, and a "load at 50% elongation" is a load (constant load) required to elongate such that an elongation ratio becomes 50%. By using a non-woven fabric or a woven fabric having a predetermined elongation recovery rate, when applied to a movable part such as a joint, the support layer expands and contracts in accordance with the movement of the application site, which is preferable.

When the support layer is a non-woven fabric, for example, the 50% elongation recovery rate of the non-woven fabric is preferably from 60 to 99%, more preferably from 65 to 95%, and still more preferably from 70 to 90%. Further, the load at 50% elongation of the non-woven fabric is preferably from 1 to 6 N/50 mm in the longitudinal direction (long-axis direction) and from 0.1 to 3 N/50 mm in the lateral direction (short-axis direction). A suitable basis weight of the support layer is, for example, from 80 to 150 g/m², and is preferably from 90 to 120 g/m². A suitable thickness of the support layer is, for example, from 0.5 to 2 mm. The basis weight and thickness of the support layer are measured in accordance with JIS L1906:2000. The stiffness of the support layer (the method for measuring the stiffness is method A (45° cantilever method) of JIS L1096:2010) can be, for example, from 20 to 40 mm in the longitudinal direction (long-axis direction) and from 10 to 35 mm in the lateral direction (short-axis direction), and is preferably from 25 to 35 mm in the longitudinal direction (long-axis direction) and from 15 to 30 mm in the lateral direction (short-axis direction).

Knitted fabrics used as the support layer include, for example, knitted fabrics processed into a cloth form by gathering meshes by circular knitting, warp (vertical) knitting, weft (horizontal) knitting, and the like. A knitted fabric made of one or a combination of two or more materials such as polyester-based, nylon-based, polypropylene-based, and rayon-based materials is preferable, and among these, a knitted fabric made of polyethylene terephthalate, which has little interaction with a drug, is more preferable.

Particularly, when the support layer is a woven fabric, the 50% elongation recovery rate of the woven fabric is, for example, preferably from 60 to 99%, more preferably from 65 to 95%, and still more preferably from 70 to 90%. Further, the load at 50% elongation is preferably from 1 to 5 N/25 mm in the longitudinal direction (long-axis direction) and from 0.1 to 3 N/25 mm in the lateral direction (short-axis direction). The stiffness of the support layer can be, for example, from 10 to 30 mm in the longitudinal direction (long-axis direction) and from 10 to 30 mm in the lateral direction (short-axis direction), and is preferably from 15 to 25 mm in the longitudinal direction (long-axis direction) and from 15 to 25 mm in the lateral direction (short-axis direction).

It is preferable that the thickness of the woven fabric as the support layer is from 0.5 to 2 mm.

Particularly, when a polyethylene terephthalate woven fabric having a basis weight of from 80 to 150 g/m² is used, water contained in the adhesive is less likely to seep out through meshes of the woven fabric at the time of spreading, and the anchoring property between the woven fabric and the adhesive is superior.

In addition, in the polyethylene terephthalate woven fabric, it is preferable that the modulus in the longitudinal direction (long-axis direction) is from 2 to 12 N/50 mm and the modulus in the lateral direction (short-axis direction) is from 2 to 8 N/50 mm (the method for measuring the modulus is in accordance with JIS L1018:1999). When the modulus is lower than 2 N/50 mm, the woven fabric stretches when the adhesive is spread, and the adhesive permeates into meshes, which may cause a reduction in the function as a gel patch. Further, when the modulus is higher than 12 N/50 mm (in the longitudinal direction) or 8 N/50 mm (in the lateral direction), extensibility is inferior, and when the gel patch is applied to a movable part, it may become difficult to follow stretching of the skin.

The material of the release liner is not particularly limited, and a liner made of a material generally known to those skilled in the art can be used. When a woven fabric, a non-woven fabric, a knitted fabric, or a resin film is used as the support layer, examples of the material of the release liner include polyethylene, polypropylene, polybutylene, polyethylene terephthalate, rayon, polyurethane, and paper, and these materials may be used singly as one kind or two or more kinds may be used in combination. The material of the release liner is preferably a polypropylene film.

The adhesive layer contains a drug, water, gelatin, *l*-menthol, a methyl acrylate/2-ethylhexyl acrylate copolymer resin emulsion, and magnesium aluminometasilicate.

The drug is not particularly limited as long as it is a drug having percutaneous absorbability. Examples of the drug include non-steroidal anti-inflammatory agents such as indomethacin, felbinac, flurbiprofen, diclofenac, diclofenac sodium, methyl salicylate, glycol salicylate, ketoprofen, and the esters thereof; antihistamines such as diphenhydramine; analgesics such as aspirin, acetaminophen, ibuprofen, and loxoprofen sodium; local anesthetics such as lidocaine; muscle relaxants such as suxamethonium chloride; antifungal agents such as clotrimazole; antihypertensive agents such as clonidine; vasodilators such as nitroglycerin and isosorbide dinitrate; vitamins such as vitamin A, vitamin E (tocopherol), tocopherol acetate, vitamin K, octotiamine, and riboflavin butyrate; prostaglandins; scopolamine; fentanyl; capsicum extract; and nonivamide. Two optical isomers, an R-form and an S-form, exist in some drugs. The drug used in the present embodiment may be any one optical isomer, or may be a mixture of two optical isomers in an arbitrary ratio. The drug may be in the form of a pharmaceutically acceptable salt, and examples of the pharmaceutically acceptable salt include inorganic basic salts such as sodium salts, potassium salts, and calcium salts; organic basic salts such as amine salts including monoethanolamine and diethanolamine; inorganic acid salts such as hydrochlorides, nitrates, and phosphates; and organic acid salts such as acetates, lactates, benzenesulfonates, and citrates.

The content of the drug in the adhesive layer may be an amount at which the drug can exert a pharmacological effect, and varies depending on the kind of the drug. For example, indomethacin can be from 0.5 % by mass to 1.5 % by mass, diclofenac sodium can be from 0.5 % by mass to 1.5 % by mass, ketoprofen can be from 0.1 % by mass to 1 % by mass, felbinac can be from 0.5 % by mass to 1.5 % by mass, and flurbiprofen can be from 0.1 % by mass to 1 % by mass.

The content of water in the adhesive layer can be from 30 % by mass to 70 % by mass based on the total mass of the adhesive layer, and may be from 30 % by mass to 60 % by mass or from 35 % by mass to 52 % by mass.

The content of gelatin in the adhesive layer is from 1 % by mass to 3 % by mass based on the total mass of the adhesive layer, and is preferably from 1.25 % by mass to 3 % by mass, more preferably from 1.5 % by mass to 3 % by mass.

The content of *l*-menthol in the adhesive layer can be from 0.5 % by mass to 3 % by mass based on the total mass of the adhesive layer, and is preferably from 1 % by mass to 2 % by mass, more preferably from 1 % by mass to 1.5 % by mass.

The methyl acrylate/2-ethylhexyl acrylate copolymer resin emulsion is an emulsion (aqueous emulsion) in which a methyl acrylate/2-ethylhexyl acrylate copolymer resin is dispersed in water. The content of the methyl acrylate/2-ethylhexyl acrylate copolymer resin in the emulsion can be from 55 % by mass to 65 % by mass based on the total mass of the emulsion. The emulsion may contain an emulsifier, and examples of the emulsifier include polyoxyethylene nonylphenyl ether. The content of the emulsifier can be from 1 % by mass to 5 % by mass based on the total mass of the emulsion. The content of the methyl acrylate/2-ethylhexyl acrylate copolymer resin emulsion in the adhesive layer is from 1.5 % by mass to 20 % by mass based on the total mass of the adhesive layer (corresponding to from 0.825 % by mass to 13 % by mass in terms of the methyl acrylate/2-ethylhexyl acrylate copolymer resin), and is preferably from 2 % by mass to 19 % by mass (corresponding to from 1.1 % by mass to 12.35 % by mass in terms of the methyl acrylate/2-ethylhexyl acrylate copolymer resin) or from 3 % by mass to 20 % by mass (corresponding to from 1.65 % by mass to 13 % by mass in terms of the methyl acrylate/2-ethylhexyl acrylate copolymer resin), and more preferably from 3 % by mass to 18 % by mass (corresponding to from 1.65 % by mass to 11.7 % by mass in terms of the methyl acrylate/2-ethylhexyl acrylate copolymer resin). Examples of commercially available methyl acrylate/2-ethylhexyl acrylate copolymer resin emulsions include NIKAZOL TS-620 (trade name, manufactured by Nippon Carbide Industries Co., Inc.) and BPW HW-2 (trade name, manufactured by TOYOCHEM Co., Ltd.).

The content of magnesium aluminometasilicate in the adhesive layer is from 0.25 % by mass to 0.45 % by mass based on the total mass of the adhesive layer, and is preferably from 0.25 % by mass to 0.43 % by mass, more preferably from 0.28 % by mass to 0.4 % by mass.

The ratio of the content of the methyl acrylate/2-ethylhexyl acrylate copolymer resin emulsion to the content of magnesium aluminometasilicate in the adhesive layer is preferably from 3.3:1 to 80:1 (corresponding to from 1.815:1 to 52:1 in terms of the methyl acrylate/2-ethylhexyl acrylate copolymer resin), more preferably from 5.4:1 to 76:1 (corresponding to from 2.97:1 to 49.4:1 in terms of the methyl acrylate/2-ethylhexyl acrylate copolymer resin) or from 6.6:1 to 80:1 (corresponding to from 3.63:1 to 52:1 in terms of the methyl acrylate/2-ethylhexyl acrylate copolymer resin), and still more preferably from 7.5:1 to 72:1 (corresponding to from 4.125:1 to 46.8:1 in terms of the methyl acrylate/2-ethylhexyl acrylate copolymer resin). The ratio of the content of the methyl acrylate/2-ethylhexyl acrylate copolymer resin emulsion to the content of gelatin in the adhesive layer is preferably from 0.5:1 to 20:1 (corresponding to from 0.275:1 to 13:1 in terms of the methyl acrylate/2-ethylhexyl acrylate copolymer resin), more preferably from 0.75:1 to 16:1 (corresponding to from 0.4125:1 to 10.4:1 in terms of the methyl acrylate/2-ethylhexyl acrylate copolymer resin) or from 1:1 to 20:1 (corresponding to from 0.55:1 to 13:1 in terms of the methyl acrylate/2-ethylhexyl acrylate copolymer resin), and still more preferably from 1:1 to 12:1 (corresponding to from 0.55:1 to 7.8:1 in terms of the methyl acrylate/2-ethylhexyl acrylate copolymer resin).

The adhesive layer may further contain, as other components, a water-soluble polymer (excluding gelatin; the same applies hereinafter), a cooling agent (excluding *l*-menthol; the same applies hereinafter), an adhesion strength enhancer (excluding the methyl acrylate/2-ethylhexyl acrylate copolymer resin emulsion; the same applies hereinafter), a dissolution aid, a cross-linking agent (excluding magnesium aluminometasilicate; the same applies hereinafter), a filler, a moisturizing agent, a stabilizer, a preservative, a chelating agent, a colorant, a fragrance agent, a pH adjusting agent, and the like. In one embodiment of the present invention, it is preferable that the adhesive layer does not contain propylene glycol.

Examples of the adhesion strength enhancer include acrylic acid ester copolymers such as aminoalkyl methacrylate copolymer E. When the adhesive layer contains the adhesion strength enhancer, the content thereof is preferably from 0.1 % by mass to 1.0 % by mass based on the total mass of the adhesive layer. It is preferable that the adhesive layer does not contain an adhesion strength enhancer other than the methyl acrylate/2-ethylhexyl acrylate copolymer resin emulsion.

The water-soluble polymer is not particularly limited as long as it can retain water in the adhesive layer, and those generally known to those skilled in the art can be used. Examples of the water-soluble polymer include polyacrylic acid, a neutralized polyacrylic acid, polyvinyl alcohol, polyvinylpyrrolidone, sodium alginate, hydroxypropyl cellulose, carboxymethylcellulose sodium (carmellose sodium), methyl cellulose, and carrageenan. The water-soluble polymer may be used singly as one kind or two or more kinds may be used in combination. The neutralized polyacrylic acid may be a completely neutralized polyacrylic acid, a partially neutralized polyacrylic acid, or a mixture thereof. Examples of the neutralized polyacrylic acid include polyacrylic acid salts, and for example, sodium salts, potassium salts, calcium salts, and ammonium salts can be used. The partially neutralized polyacrylic acid is one in which structural units derived from acrylic acid and structural units derived from an acrylic acid salt are present in an arbitrary ratio in one polymer chain. It is preferable to use a partially neutralized polyacrylic acid in which 50 mol% of the carboxyl groups in one polymer chain are neutralized. It is preferable that the water-soluble polymer contains at least one selected from the group consisting of polyacrylic acid and a neutralized polyacrylic acid, and it is more preferable that the water-soluble polymer contains polyacrylic acid and a neutralized polyacrylic acid. When the adhesive layer contains the water-soluble polymer, the content thereof is preferably from 3 % by mass to 15 % by mass based on the total mass of the adhesive layer, more preferably from 5 % by mass to 13 % by mass, and still more preferably from 7 % by mass to 11 % by mass. When the adhesive layer contains polyacrylic acid, the content thereof is preferably from 1 % by mass to 5 % by mass based on the total mass of the adhesive layer, more preferably from 2 % by mass to 5 % by mass, and still more preferably from 2.5 % by mass to 4.5 % by mass. When the adhesive layer contains a neutralized polyacrylic acid, the content thereof is preferably from 1 % by mass to 5 % by mass based on the total mass of the adhesive layer, more preferably from 1.5 % by mass to 4.5 % by mass, and still more preferably from 2 % by mass to 4 % by mass. When the adhesive layer contains polyvinyl alcohol, the content thereof is preferably from 1 % by mass to 5 % by mass based on the total mass of the adhesive layer, more preferably from 1 % by mass to 4 % by mass, and still more preferably from 1.5 % by mass to 3.5 % by mass.

Examples of the cooling agent include thymol, *l*-isopulegol, and peppermint oil. The cooling agent may be used singly as one kind or two or more kinds may be used in combination. When the adhesive layer contains the cooling agent, the content thereof is preferably from 1 % by mass to 1.5 % by mass based on the total mass of the adhesive layer.

The dissolution aid is not particularly limited as long as it can dissolve a drug, and examples thereof include crotamiton; N-methylpyrrolidone; polyalkylene glycols such as polyethylene glycol (PEG) and polybutylene glycol; oxyalkylene fatty acid esters such as polyethylene glycol monostearate; polyoxyalkylene sorbitan fatty acid esters such as polysorbate 80; sorbitan fatty acid esters such as sorbitan monooleate; and surfactants such as polyoxyethylene hardened castor oil. These dissolution aids may be used singly as one kind or two or more kinds may be used in combination. When the adhesive layer contains the dissolution aid, the content thereof is preferably from 0.1 % by mass to 3 % by mass based on the total mass of the adhesive layer, more preferably from 0.2 % by mass to 1 % by mass, and still more preferably from 0.3 % by mass to 0.7 % by mass.

The cross-linking agent is a component for adjusting the degree of progress of a cross-linking reaction between neutralized polyacrylic acids and of a cross-linking reaction between a neutralized polyacrylic acid and a polyacrylic acid optionally added, and a cross-linking agent generally used in gel patches can be used. Examples of the cross-linking agent include aluminum compounds. When the adhesive layer contains the cross-linking agent, the content thereof is preferably from 0.05 % by mass to 1 % by mass based on the total mass of the adhesive layer, more preferably from 0.1 % by mass to 0.7 % by mass, and still more preferably from 0.2 % by mass to 0.5 % by mass. When the content of the cross-linking agent is within the above range, a gel patch having superior followability to the skin can be obtained. It is preferable that the adhesive layer does not contain a cross-linking agent other than magnesium aluminometasilicate.

Examples of the filler include inorganic fillers such as anhydrous silicic acid, titanium oxide, synthetic aluminum silicate, kaolin, talc, bentonite, and magnesium aluminometasilicate, and any one of these or a combination of two or more of these may be used. When the adhesive layer contains the filler, the content thereof is preferably from 0.1 % by mass to 1.5 % by mass based on the total mass of the adhesive layer, more preferably from 0.2 % by mass to 1 % by mass, and still more preferably from 0.3 % by mass to 0.7 % by mass.

The moisturizing agent is not particularly limited as long as it can suppress evaporation of water from the adhesive layer with the passage of time. Examples of the moisturizing agent include polyhydric alcohols such as glycerin, sorbitol, ethylene glycol, 1,4-butanediol, polyethylene glycol, and liquid paraffin. These moisturizing agents may be used singly as one kind or two or more kinds may be used in combination. It is preferable that the moisturizing agent contains at least one selected from the group consisting of glycerin and sorbitol, and it is more preferable that the moisturizing agent contains glycerin and sorbitol. When the adhesive layer contains the moisturizing agent, the content thereof is preferably from 15 % by mass to 45 % by mass based on the total mass of the adhesive layer, more preferably from 20 % by mass to 40 % by mass, and still more preferably from 25 % by mass to 35 % by mass.

Examples of the stabilizer include oxybenzone, dibutylhydroxytoluene (BHT), edetate sodium, and UV absorbers (for example, dibenzoylmethane derivatives). When the adhesive layer contains the stabilizer, the content thereof is preferably from 0.05 % by mass to 1 % by mass based on the total mass of the adhesive layer, more preferably from 0.1 % by mass to 0.6 % by mass, and still more preferably from 0.2 % by mass to 0.6 % by mass.

Examples of the preservative include methyl p-hydroxybenzoate, ethyl p-hydroxybenzoate, propyl p-hydroxybenzoate, butyl p-hydroxybenzoate, 1,2-pentanediol, benzoic acid and salts thereof, salicylic acid and salts thereof, sorbic acid and salts thereof, dehydroacetic acid and salts thereof, 4-isopropyl-3-methylphenol, 2-isopropyl-5-methylphenol, phenol, hinokitiol, cresol, 2,4,4'-trichloro-2'-hydroxydiphenyl ether, 3,4,4'-trichlorocarbanilide, chlorobutanol, benzalkonium chloride, and benzethonium chloride. These preservatives may be used singly as one kind or two or more kinds may be used in combination. When the adhesive layer contains the preservative, the content thereof is preferably from 0.01 % by mass to 1 % by mass based on the total mass of the adhesive layer, more preferably from 0.05 % by mass to 0.5 % by mass, and still more preferably from 0.1 % by mass to 0.2 % by mass.

Examples of the chelating agent include edetic acid (also known as ethylenediaminetetraacetic acid, EDTA) or a salt thereof, ascorbic acid, pyrophosphate, hexametaphosphate, gluconate, and the like. These chelating agents may be used singly as one kind or two or more kinds may be used in combination. A more specific example of the edetic acid salt is edetate sodium. When the adhesive layer contains the chelating agent, the content thereof is preferably from 0.05 % by mass to 1 % by mass based on the total mass of the adhesive layer, more preferably from 0.1 % by mass to 0.6 % by mass, and still more preferably from 0.2 % by mass to 0.6 % by mass.

The total mass of the adhesive layer can be from 200 g/m² to 1000 g/m², may be from 400 g/m² to 1000 g/m², and may be from 400 g/m² to 650 g/m². It is preferable that the total mass is from 400 g/m² to 650 g/m², whereby the feeling of fit is improved and adhesion over a longer period can be improved. When the total mass of the adhesive layer is within the above range, the thickness of the entire gel patch can be reduced, whereby it easily follows the skin, and further, when attached, a step between the periphery and the surrounding portion becomes small, so that there is a tendency that peeling is less likely to occur.

The pH of the adhesive layer is preferably from 4 to 8, and more preferably from 4.5 to 6. By setting the pH to 4 or more, irritation to the skin is reduced, and by setting the pH to 8 or less, the moldability and shape retention of the gel patch can be improved. Particularly, when the support layer is a woven fabric, especially a knitted fabric, seepage may occur when the adhesive layer is formed, but when the pH is from 5 to 6.5, there is a tendency that seepage is suppressed. The pH can be measured, for example, in accordance with the pH measurement method of the General Tests of the Japanese Pharmacopoeia by using a glass combination electrode and measuring after diluting a sample 20-fold with purified water.

The gel patch can be produced, for example, by mixing a drug, water, gelatin, *l*-menthol, a methyl acrylate/2-ethylhexyl acrylate copolymer resin emulsion, magnesium aluminometasilicate, and the optional components described above as needed to obtain an adhesive composition; uniformly spreading this adhesive composition on a release liner; and laminating a support layer thereon. The gel patch may also be produced by uniformly spreading the adhesive composition on the support layer and laminating the release liner thereon.

### Examples

An adhesive composition was prepared by mixing the respective components in the compositions (the numerical values of the respective components in the tables represent % by mass) shown in Examples and Comparative Examples in Tables 1 to 4. Then, the obtained adhesive composition was uniformly spread on a release liner (polypropylene film) so that the mass of the adhesive layer per unit area became 500 g/m², and a support layer (polyethylene terephthalate non-woven fabric, basis weight: 100 g/m²) was laminated thereon, followed by cutting into dimensions of 140 cm² (10 cm × 14 cm) to obtain gel patches. As the methyl acrylate/2-ethylhexyl acrylate copolymer resin emulsion, NIKAZOL TS-620 (trade name, manufactured by Nippon Carbide Industries Co., Inc.) was used. In addition, concentrated glycerin and D-sorbitol solution described in the Japanese Pharmacopoeia (The Japanese Pharmacopoeia, Eighteenth Edition) were used. Each gel patch was evaluated for *l*-menthol precipitation, unevenness at the time of spreading, seepage, liner shift, liner releasability, initial adhesiveness, time-dependent adhesiveness, pain at the time of peeling, and adhesive residue according to the following methods.

### <Unevenness at the Time of Spreading>

The adhesive layer at the time of spreading was observed and evaluated in accordance with the following evaluation scores. A score of 2 or more was determined to be acceptable.

2. The surface and thickness are uniform over the entire area (area: 90% or more) of the adhesive layer, and there is no spreading unevenness.
1. In a part (area: more than 10%) of the adhesive layer, the surface and/or thickness is not uniform, and spreading unevenness is observed.

In all of the following evaluations, gel patches were sealed in aluminum packaging bags, allowed to stand at 25°C for 10 days or more and up to 15 days, and then evaluated on gel patches taken out from the packaging bags.

### < l-Menthol Precipitation>

The surface of the adhesive layer of the gel patch was visually observed, and the presence or absence of precipitation of *l*-menthol crystals was evaluated according to the following criteria. Ten gel patches were evaluated, and the average value of the scores was adopted as the evaluation result, and a score (average value) of 2.0 or more was determined to be acceptable.

### 2. No precipitation

### 1. Precipitation present

### <Seepage>

The area of a portion where components of the adhesive layer had seep seeped out into the support layer was measured and evaluated in accordance with the following evaluation scores. Five to twenty gel patches were evaluated, and the average value of the scores was adopted as the evaluation result, and a score (average value) of 5.0 or more was determined to be acceptable.
5. No seepage
4. Slight seepage (area: less than 10%)
3. Some seepage (area: 10% or more and less than 20%)
2. Much seepage (area: 20% or more and less than 30%)
1. Considerable seepage (area: 30% or more)

### <Liner Shift>

The degree of shift between the adhesive layer and the release liner of the gel patch was scored according to the following criteria. Five to twenty gel patches were evaluated, and the average value of the scores was adopted as the evaluation result, and a score (average value) of 4.0 or more was determined to be acceptable.
4. No liner shift (shift of less than 0.5 mm)
3. Almost no liner shift (shift of 0.5 mm or more and less than 1.5 mm)
2. Apparent liner shift (shift of 1.5 mm or more)
1. Measurement impossible (liner layer fell off during the test)

### <Liner Releasability>

The easiness of peeling off and removing the release liner from the gel patch was scored according to the following criteria. Five to twenty gel patches were evaluated, and the average value of the scores was adopted as the evaluation result, and a score (average value) of 3.0 or more was determined to be acceptable.
5. Very easy to peel
4. Somewhat easy to peel
3. Acceptable releasability
2. Somewhat difficult to peel
1. Very difficult to peel

### <Initial Adhesiveness>

The release liner was peeled and removed from the gel patch, the gel patch was applied to the elbow of each of 5 to 20 subjects, and the elbow was bent 40 times, and then the adhesiveness of the adhesive layer to the skin was scored according to the following criteria. The average value of the scores of the 5 to 20 subjects was adopted as the evaluation result, and a score (average value) of 5.0 or more was determined to be acceptable.
6. No peeling
5. Peeling at an edge (less than 1/4)
4. Peeling of 1/4 or more and less than 1/3
3. Peeling of 1/3 or more and less than 1/2
2. Peeling of 1/2 or more
1. Fallen off

### <Time-Dependent Adhesiveness>

The release liner was peeled and removed from the gel patch, the gel patch was applied to the elbow of each of 5 to 20 subjects, and the adhesiveness of the adhesive layer to the skin after 4 hours and after 6 hours was scored according to the following criteria, respectively. The average value of the scores of the 5 to 20 subjects was adopted as the evaluation result, and a score (average value) of 5.0 or more was determined to be acceptable.
6. No peeling
5. Peeling at an edge (less than 1/4)
4. Peeling of 1/4 or more and less than 1/3
3. Peeling of 1/3 or more and less than 1/2
2. Peeling of 1/2 or more
1. Fallen off

### <Pain at the Time of Peeling>

In the above time-dependent adhesiveness test, after 6 hours of application, the gel patch was peeled from the elbow of each subject, and pain at the time of peeling the gel patch was scored according to the following criteria. The average value of the scores of the 5 to 20 subjects was adopted as the evaluation result, and a score (average value) of 3.0 or more was determined to be acceptable.
4. None
3. Slight (degree that is not bothersome)
2. Some
1. Considerable

### <Adhesive Residue>

In the above pain-at-the-time-of-peeling test, the residual ratio (on an area basis) of the adhesive layer on the skin after peeling the gel patch was scored according to the following criteria. The average value of the scores of the 5 to 20 subjects was adopted as the evaluation result, and a score (average value) of 3.0 or more was determined to be acceptable.
4. None
3. Slight residue (less than 1/10)
2. Minor residue (1/10 or more and less than 1/5)
1. Considerable residue (1/5 or more)

The results are summarized in Tables 1 to 4. In the tables, A indicates that the acceptable conditions in the above evaluation are satisfied, and B indicates that the acceptable conditions are not satisfied.

**[Table 1]**

| | Comp. Ex. 1 | Comp. Ex. 2 | Comp. Ex. 3 | Comp. Ex. 4 | Comp. Ex. 5 | Comp. Ex. 6 |
|---|---|---|---|---|---|---|
| Indomethacin | 1.0 | 1.0 | 1.0 | 1.0 | 1.0 | 1.0 |
| 1-menthol | 1.2 | 1.2 | 1.2 | 1.2 | 1.2 | 1.2 |
| methyl acrylate/2-ethylhexyl acrylate copolymer resin emulsion | 0.50 | 3.0 | 18 | 3.0 | 18 | 25 |
| Magnesium aluminometasilicate | 0.30 | 0.23 | 0.23 | 0.80 | 0.80 | 0.30 |
| Gelatin | 2.4 | 2.4 | 2.4 | 1.5 | 1.5 | 2.4 |
| Partially neutralized polyacrylic acid | 3.0 | 3.0 | 3.0 | 3.0 | 3.0 | 3.0 |
| Polyacrylic acid | 3.5 | 3.5 | 3.5 | 3.5 | 3.5 | 3.5 |
| Polyvinyl alcohol | 2.6 | 2.6 | 2.6 | 2.6 | 2.6 | 2.6 |
| Concentrated glycerin | 23.0 | 23.0 | 23.0 | 23.0 | 23.0 | 23.0 |
| D-sorbitol solution | 7.5 | 7.5 | 7.5 | 7.5 | 7.5 | 7.5 |
| Edetate sodium | 0.4 | 0.4 | 0.4 | 0.4 | 0.4 | 0.4 |
| Others | 1.15 | 1.15 | 1.15 | 1.15 | 1.15 | 1.15 |
| Total amount (Adjusted to 100 with water) | 100 | 100 | 100 | 100 | 100 | 100 |
| 1-menthol precipitation | B(1.0) | A(2.0) | A(2.0) | A(2.0) | A(2.0) | A(2.0) |
| Unevenness at the time of spreading | A(2) | A(2) | A(2) | A(2) | A(2) | A(2) |
| Seepage | A(5.0) | A(5.0) | B(4.9) | B(4.9) | B(4.0) | A(5.0) |
| Liner shift | A(4.0) | A(4.0) | A(4.0) | A(4.0) | A(4.0) | A(4.0) |
| Liner releasability | B(1.67) | B(1.9) | A(3.6) | A(4.1) | A(4.0) | A(4.0) |
| Initial adhesiveness | A(5.8) | A(5.8) | A(5.8) | B(4.4) | B(4.5) | A(5.6) |
| Time-dependent adhesiveness (4h) | A(5.4) | A(5.6) | A(5.7) | B(3.9) | B(4.9) | A(5.4) |
| Time-dependent adhesiveness (6h) | A(4.8) | A(5.5) | A(5.7) | B(3.9) | B(4.9) | A(5.2) |
| Pain at the time of peeling | A(3.7) | A(3.7) | A(3.2) | A(3.9) | A(3.8) | B(2.8) |
| Adhesive Residue | A(4.0) | A(3.5) | A(3.6) | A(4.0) | A(4.0) | A(3.9) |

As is apparent from the results shown in Table 1, it was confirmed that, in a gel patch (Comparative Example 1) in which the content of the methyl acrylate/2-ethylhexyl acrylate copolymer resin emulsion is less than 1.5 % by mass, crystals of *l*-menthol precipitate and liner releasability is insufficient. On the other hand, in gel patches (Comparative Examples 2 to 6) in which the content of the methyl acrylate/2-ethylhexyl acrylate copolymer resin emulsion is from 3.0 % by mass to 25 % by mass, although precipitation of *l*-menthol crystals is suppressed, it was confirmed that another problem arises in that at least one of liner releasability, adhesiveness, seepage, and pain at the time of peeling becomes insufficient.

**[Table 2]**

| | Comp. Ex. 1 | Comp. Ex. 7 | Comp. Ex. 8 | Comp. Ex. 9 | Comp. Ex. 10 |
|---|---|---|---|---|---|
| Indomethacin | 1.0 | 1.0 | 1.0 | 1.0 | 1.0 |
| 1-menthol | 1.2 | 1.2 | 1.2 | 1.2 | 1.2 |
| methyl acrylate/2-ethylhexyl acrylate copolymer resin emulsion | 0.50 | 3.0 | 3.0 | 3.0 | 3.0 |
| Magnesium aluminometasilicate | 0.30 | 0.10 | 0.20 | 0.30 | 0.30 |
| Gelatin | 2.4 | 6.0 | 3.5 | 4.0 | 0.90 |
| Partially neutralized polyacrylic acid | 3.0 | 3.0 | 3.0 | 3.0 | 3.0 |
| Polyacrylic acid | 3.5 | 3.5 | 3.5 | 3.5 | 3.5 |
| Polyvinyl alcohol | 2.6 | 2.6 | 2.6 | 2.6 | 2.6 |
| Concentrated glycerin | 23.0 | 23.0 | 23.0 | 23.0 | 23.0 |
| D-sorbitol solution | 7.5 | 7.5 | 7.5 | 7.5 | 7.5 |
| Edetate sodium | 0.4 | 0.4 | 0.4 | 0.4 | 0.4 |
| Others | 1.15 | 1.15 | 1.15 | 1.15 | 1.15 |
| Total amount (Adjusted to 100 with water) | 100 | 100 | 100 | 100 | 100 |
| l-menthol precipitation | B(1.0) | - | - | A(2.0) | A(2.0) |
| Unevenness at the time of spreading | A(2) | B(1) | B(1) | A(2) | A(2) |
| Seepage | A(5.0) | - | - | A(5.0) | B(4.8) |
| Liner shift | A(4.0) | - | - | A(4.0) | A(4.0) |
| Liner releasability | B(1.67) | - | | A(3.5) | B(2.9) |
| Initial adhesiveness | A(5.8) | - | - | A(5.5) | A(6.0) |
| Time-dependent adhesiveness (4h) | A(5.4) | - | - | A(5.1) | A(5.6) |
| Time-dependent adhesiveness (6h) | A(4.8) | - | - | B(4.7) | A(5.4) |
| Pain at the time of peeling | A(3.7) | - | - | A(3.8) | A(3.5) |
| Adhesive Residue | A(4.0) | - | - | A(3.9) | A(3.8) |

| | Comp. Ex. 11 | Comp. Ex. 12 | Comp. Ex. 13 | Comp. Ex. 14 | Comp. Ex. 15 |
|---|---|---|---|---|---|
| Indomethacin | 1.0 | 1.0 | 1.0 | 1.0 | 1.0 |
| 1-menthol | 1.2 | 1.2 | 1.2 | 1.2 | 1.2 |
| methyl acrylate/2-ethylhexyl acrylate copolymer resin emulsion | 3.0 | 18 | 18 | 18 | 18 |
| Magnesium aluminometasilicate | 0.50 | 0.20 | 0.30 | 0.30 | 0.50 |
| Gelatin | 3.5 | 3.5 | 4.0 | 0.9 | 3.5 |
| Partially neutralized polyacrylic acid | 3.0 | 3.0 | 3.0 | 3.0 | 3.0 |
| Polyacrylic acid | 3.5 | 3.5 | 3.5 | 3.5 | 3.5 |
| Polyvinyl alcohol | 2.6 | 2.6 | 2.6 | 2.6 | 2.6 |
| Concentrated glycerin | 23.0 | 23.0 | 23.0 | 23.0 | 23.0 |
| D-sorbitol solution | 7.5 | 7.5 | 7.5 | 7.5 | 7.5 |
| Edetate sodium | 0.4 | 0.4 | 0.4 | 0.4 | 0.4 |
| Others | 1.15 | 1.15 | 1.15 | 1.15 | 1.15 |
| Total amount (Adjusted to 100 with water) | 100 | 100 | 100 | 100 | 100 |
| l-menthol precipitation | A(2.0) | - | A(2.0) | A(2.0) | A(2.0) |
| Unevenness at the time of spreading | A(2) | B(1) | A(2) | A(2) | B(1) |
| Seepage | B(4.9) | - | B(4.9) | B(4.9) | B(4.0) |
| Liner shift | A(4.0) | - | A(4.0) | A(4.0) | A(4.0) |
| Liner releasability | A(3.9) | - | A(4.3) | A(3.5) | A(4.3) |
| Initial adhesiveness | A(5.3) | - | B(4.9) | A(5.8) | B(4.6) |
| Time-dependent adhesiveness (4h) | A(5.1) | - | A(5.2) | A(5.6) | A(5.1) |
| Time-dependent adhesiveness (6h) | B(4.3) | - | A(5.1) | A(5.5) | A(5.0) |
| Pain at the time of peeling | A(4.0) | - | A(3.6) | A(3.3) | A(3.6) |
| Adhesive Residue | A(4.0) | - | A(3.9) | A(3.8) | A(4.0) |

| | | | | | |
|---|---|---|---|---|---|
| "-" indicates evaluation was not performed because formulation was impossible. | | | | | |

As is apparent from the results shown in Table 2, it was confirmed that, in gel patches (Comparative Examples 7, 8, 11, 12, and 15) in which the content of magnesium aluminometasilicate is less than 0.25 % by mass or more than 0.45 % by mass and in gel patches (Comparative Examples 7 to 15) in which the content of gelatin is less than 1 % by mass or more than 3 % by mass, at least one of spreading unevenness at the time of spreading, adhesiveness, seepage, and liner releasability is insufficient.

**[Table 3]**

| | Ex. 1 | Ex. 2 | Ex. 3 | Ex. 4 |
|---|---|---|---|---|
| Indomethacin | 1.0 | 1.0 | 1.0 | 1.0 |
| 1-menthol | 1.2 | 1.2 | 1.2 | 1.2 |
| methyl acrylate/2-ethylhexyl acrylate copolymer resin emulsion | 3.0 | 3.0 | 3.0 | 7.8 |
| Magnesium aluminometasilicate | 0.28 | 0.30 | 0.40 | 0.30 |
| Gelatin | 3.0 | 2.4 | 1.5 | 2.4 |
| Partially neutralized polyacrylic acid | 3.0 | 3.0 | 3.0 | 3.0 |
| Polyacrylic acid | 3.5 | 3.5 | 3.5 | 3.5 |
| Polyvinyl alcohol | 2.6 | 2.6 | 2.6 | 2.6 |
| Concentrated glycerin | 23.0 | 23.0 | 23.0 | 23.0 |
| D-sorbitol solution | 7.5 | 7.5 | 7.5 | 7.5 |
| Edetate sodium | 0.4 | 0.4 | 0.4 | 0.4 |
| Others | 1.15 | 1.15 | 1.15 | 1.15 |
| Total amount (Adjusted to 100 with water) | 100 | 100 | 100 | 100 |
| 1-menthol precipitation | A(2.0) | A(2.0) | A(2.0) | A(2.0) |
| Unevenness at the time of spreading | A(2) | A(2) | A(2) | A(2) |
| Seepage | A(5.0) | A(5.0) | A(5.0) | A(5.0) |
| Liner shift | A(4.0) | A(4.0) | A(4.0) | A(4.0) |
| Liner releasability | A(3.0) | A(3.0) | A(3.6) | A(3.1) |
| Initial adhesiveness | A(5.8) | A(5.8) | A(5.8) | A(5.9) |
| Time-dependent adhesiveness (4h) | A(5.5) | A(5.6) | A(5.4) | A(5.8) |
| Time-dependent adhesiveness (6h) | A(5.1) | A(5.4) | A(5.1) | A(5.7) |
| Pain at the time of peeling | A(3.8) | A(3.3) | A(3.6) | A(3.1) |
| Adhesive Residue | A(3.9) | A(4.0) | A(4.0) | A(3.6) |

| | Ex. 5 | Ex. 6 | Ex. 7 | Ex. 8 |
|---|---|---|---|---|
| Indomethacin | 1.0 | 1.0 | 1.0 | 1.0 |
| 1-menthol | 1.2 | 1.2 | 1.2 | 1.2 |
| methyl acrylate/2-ethylhexyl acrylate copolymer resin emulsion | 13 | 18 | 18 | 18 |
| Magnesium aluminometasilicate | 0.30 | 0.25 | 0.30 | 0.40 |
| Gelatin | 2.4 | 3.0 | 2.4 | 1.5 |
| Partially neutralized polyacrylic acid | 3.0 | 3.0 | 3.0 | 3.0 |
| Polyacrylic acid | 3.5 | 3.5 | 3.5 | 3.5 |
| Polyvinyl alcohol | 2.6 | 2.6 | 2.6 | 2.6 |
| Concentrated glycerin | 23.0 | 23.0 | 23.0 | 23.0 |
| D-sorbitol solution | 7.5 | 7.5 | 7.5 | 7.5 |
| Edetate sodium | 0.4 | 0.4 | 0.4 | 0.4 |
| Others | 1.15 | 1.15 | 1.15 | 1.15 |
| Total amount (Adjusted to 100 with water) | 100 | 100 | 100 | 100 |
| l-menthol precipitation | A(2.0) | A(2.0) | A(2.0) | A(2.0) |
| Unevenness at the time of spreading | A(2) | A(2) | A(2) | A(2) |
| Seepage | A(5.0) | A(5.0) | A(5.0) | A(5.0) |
| Liner shift | A(4.0) | A(4.0) | A(4.0) | A(4.0) |
| Liner releasability | A(3.3) | A(4.6) | A(4.0) | A(3.8) |
| Initial adhesiveness | A(5.8) | A(5.6) | A(5.8) | A(5.6) |
| Time-dependent adhesiveness (4h) | A(5.8) | A(5.4) | A(5.8) | A(5.7) |
| Time-dependent adhesiveness (6h) | A(5.6) | A(5.1) | A(5.8) | A(5.7) |
| Pain at the time of peeling | A(3.3) | A(3.6) | A(3.0) | A(3.0) |
| Adhesive Residue | A(4.0) | A(3.7) | A(3.8) | A(4.0) |

**[Table 4]**

| | Ex. 14 | Ex. 15 | Ex. 16 | Ex. 17 | Ex. 18 |
|---|---|---|---|---|---|
| Indomethacin | 1.0 | 1.0 | 1.0 | 1.0 | 1.0 |
| 1-menthol | 3.0 | 0.5 | 1.2 | 1.2 | 1.2 |
| methyl acrylate/2-ethylhexyl acrylate copolymer resin emulsion | 3.0 | 7.8 | 7.8 | 7.8 | 20 |
| Magnesium aluminometasilicate | 0.30 | 0.30 | 0.30 | 0.45 | 0.30 |
| Gelatin | 2.4 | 2.4 | 1 | 2.4 | 2.4 |
| Partially neutralized polyacrylic acid | 3.0 | 3.0 | 3.0 | 3.0 | 3.0 |
| Polyacrylic acid | 3.5 | 3.5 | 3.5 | 3.5 | 3.5 |
| Polyvinyl alcohol | 2.6 | 2.6 | 2.6 | 2.6 | 2.6 |
| Concentrated glycerin | 23.0 | 23.0 | 23.0 | 23.0 | 23.0 |
| D-sorbitol solution | 7.5 | 7.5 | 7.5 | 7.5 | 7.5 |
| Edetate sodium | 0.4 | 0.4 | 0.4 | 0.4 | 0.4 |
| Others | 1.15 | 1.15 | 1.15 | 1.15 | 1.15 |
| Total amount (Adjusted to 100 with water) | 100 | 100 | 100 | 100 | 100 |
| 1-menthol precipitation | A(2.0) | A(2.0) | A(2.0) | A(2.0) | A(2.0) |
| Unevenness at the time of spreading | A(2) | A(2) | A(2) | A(2) | A(2) |
| Seepage | A(5.0) | A(5.0) | A(5.0) | A(5.0) | A(5.0) |
| Liner shift | A(4.0) | A(4.0) | A(4.0) | A(4.0) | A(4.0) |
| Liner releasability | A(3.0) | A(3.3) | A(3.0) | A(3.9) | A(3.4) |
| Initial adhesiveness | A(5.6) | A(5.6) | A(5.9) | A(5.3) | A(5.8) |
| Time-dependent adhesiveness (4h) | A(5.4) | A(5.5) | A(5.4) | A(5.0) | A(5.6) |
| Time-dependent adhesiveness (6h) | A(5.0) | A(5.4) | A(5.1) | A(5.0) | A(5.3) |
| Pain at the time of peeling | A(3.5) | A(3.0) | A(3.0) | A(3.4) | A(3.0) |
| Adhesive Residue | A(4.0) | A(4.0) | A(3.9) | A(4.0) | A(3.9) |

As is apparent from the results shown in Tables 3 and 4, it was confirmed that the gel patches (Examples 1 to 8 and 14 to 18) of the present invention are excellent in all evaluation items.

Furthermore, gel patches (Examples 9 to 12) were produced in the same manner as in Example 4 except that diclofenac sodium (1.0 % by mass), ketoprofen (0.3 % by mass), felbinac (0.7 % by mass), or flurbiprofen (0.33 % by mass) was used instead of indomethacin, and the gel patches were evaluated. The gel patches of Examples 9 to 12 all showed good results in all evaluation items.

Furthermore, a gel patch (Example 13) was produced in the same manner as in Example 4 except that BPW HW-2 (trade name, manufactured by TOYOCHEM Co., Ltd.) was used as the methyl acrylate/2-ethylhexyl acrylate copolymer resin emulsion instead of NIKAZOL TS-620 (trade name, manufactured by Nippon Carbide Industries Co., Inc.), and the gel patch was evaluated. The gel patch of Example 13 showed good results in all evaluation items.

## Claims

1. A gel patch comprising:
a support layer;
an adhesive layer laminated on the support layer; and
a release liner provided on a surface of the adhesive layer opposite to the support layer,
wherein the adhesive layer comprises a drug, water, gelatin, *l-*menthol, a methyl acrylate/2-ethylhexyl acrylate copolymer resin emulsion, and magnesium aluminometasilicate, and
wherein based on the total mass of the adhesive layer, the content of the methyl acrylate/2-ethylhexyl acrylate copolymer resin emulsion is from 1.5 % by mass to 20 % by mass, the content of the magnesium aluminometasilicate is from 0.25 % by mass to 0.45 % by mass, and the content of the gelatin is from 1 % by mass to 3 % by mass.

2. The gel patch according to claim 1, wherein the adhesive layer further comprises at least one selected from the group consisting of polyacrylic acid and a neutralized polyacrylic acid.
